(19) Europäisches Patentamt / European Patent Office / Office européen des brevets

(11) **EP 2 471 441 B1**

(12) **EUROPÄISCHE PATENTSCHRIFT**

(45) Veröffentlichungstag und Bekanntmachung des Hinweises auf die Patenterteilung:
**16.08.2017 Patentblatt 2017/33**

(51) Int Cl.:
*A61B 3/10* *(2006.01)*      *A61B 3/11* *(2006.01)*
*A61B 3/113* *(2006.01)*

(21) Anmeldenummer: **12002380.9**

(22) Anmeldetag: **04.02.2010**

(54) **Verfahren und Vorrichtung zur Bestimmung der Augendrehpunktlage**

Device and method for determining the eye's centre of rotation

Procédé et dispositif destinés à la détermination de la position du point rotatif oculaire

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **26.02.2009 DE 102009010467**

(43) Veröffentlichungstag der Anmeldung:
**04.07.2012 Patentblatt 2012/27**

(60) Teilanmeldung:
**17175172.0**
**17178300.4**

(62) Dokumentnummer(n) der früheren Anmeldung(en) nach Art. 76 EPÜ:
**10704094.1 / 2 400 880**

(73) Patentinhaber: **Carl Zeiss Vision GmbH**
**73430 Aalen (DE)**

(72) Erfinder:
• **Kratzer, Timo**
**73434 Aalen (DE)**

• **Cabeza-Guillèn, Jesús-Miguel**
**73434 Aalen (DE)**
• **Kelch, Gerhard**
**73434 Aalen (DE)**

(74) Vertreter: **Carl Zeiss AG - Patentabteilung**
**Patentabteilung / CLP-P**
**Carl-Zeiss-Strasse 22**
**73447 Oberkochen (DE)**

(56) Entgegenhaltungen:
**EP-A1- 1 154 302**      **EP-A1- 1 837 699**
**EP-A2- 0 350 957**      **JP-A- 2004 008 768**

• **THIBOS L N ET AL: "Clinical applications of the Shack-Hartmann aberrometer.", OPTOMETRY AND VISION SCIENCE : OFFICIAL PUBLICATION OF THE AMERICAN ACADEMY OF OPTOMETRY DEC 1999, Bd. 76, Nr. 12, Dezember 1999 (1999-12), Seiten 817-825, XP002575578, ISSN: 1040-5488**

**Beschreibung**

Die Erfindung bezieht sich auf ein Verfahren, ein Computerprogramm und eine Vorrichtung zur Bestimmung der Augendrehpunktlage. Weiterhin offenbart wird ein Verfahren zum Optimieren eines für ein Auge individuellen Brillenglases, bei dem die Augendrehpunktlage ermittelt und als Eingangsparameter verwendet wird.

Es ist bekannt, zum Optimieren von Brillengläsern, insbesondere von individuellen Gleitsichtgläsern, verschiedene Parameter des Systems Auge/Brille zu berücksichtigen. Diese Parameter sind beispielsweise der Pupillenabstand PD, der Hornhaut-Scheitelabstand HSA, der Vorneigungswinkel $\alpha$ des Brillenglases, die Fassungsscheibenwinkel $\alpha_R$, $\alpha_L$ für das rechte und linke Brillenglas sowie die Lage des optischen oder mechanischen Augendrehpunkts Z', M. Unter individuellen Gleitsichtgläsern versteht man Gleitsichtgläser, bei denen mindestens ein individueller Gebrauchsparameter bei der Berechnung des Brillenglases berücksichtigt wird und die mittels Freiformtechnologie gefertigt werden, wie dies z.B. in der Deutschen Optikerzeitung DOZ 4 + 5/2000 von W. Grimm und G. Kelch in dem Aufsatz "Gradal® Individual: Konzeption, Fertigung und Anpassung", in der EP 857 993 B1 und/oder der EP 562 336 B1 beschrieben ist. Bei nicht-individuellen Brillengläsern werden diese Parameter aus statistischen Mittelwerten eines repräsentativen Bevölkerungsquerschnitts ermittelt. Bei individuellen Brillengläsern hingegen werden die Parameter individuell an dem jeweiligen Brillenträger gemessen, beispielsweise mit so genannten Videozentriersystemen, wie sie von der Anmelderin unter den Typenbezeichnungen "i.Terminal" und "Relaxed Vision Terminal" hergestellt und vertrieben werden. Diese Videozentriersysteme sind jedoch bisher nicht in der Lage, eine Optimierung des Brillenglases mit Hilfe der Augendrehpunktlage in einer ausreichend exakten Weise zu ermöglichen.

Wenn man die Augendrehpunktlage berücksichtigen möchte, kann man diese in bekannter Weise aus der Augenlänge über die mittlere Sphäre des verordneten Brillenglases ableiten. Dabei werden allerdings viele Annahmen gemacht, die mit den tatsächlichen Gegebenheiten nicht ausreichend übereinstimmen.

Der Zusammenhang zwischen Augenlänge und der Sphäre des verordneten Brillenglases wird oft vereinfacht als linear angenommen. Dies ist aber in der Realität nicht der Fall, weil sowohl die Krümmung der Hornhaut als auch die Augenlinse sowie die Augenlänge teilweise sehr unabhängig voneinander wachsen bzw. sich unterschiedlich entwickeln.

Im Allgemeinen ist es ausreichend, den Augendrehpunkt als punktförmiges Drehzentrum innerhalb des Auges zu betrachten. Die Erfindung umfasst jedoch ganz allgemein auch ein ausgedehntes, im Allgemeinen näherungsweise kugelförmiges Augendrehpunktsgebiet. Zur Beschreibung der Erfindung und des Standes der Technik wird nachfolgend der Einfachheit halber von einem punktförmigen Augendrehpunkt ausgegangen, soweit nichts Anderes erwähnt ist.

Üblicherweise geht man bei der Verordnung einer Brille so vor, dass die Brillengläser auf der Grundlage einer subjektiven Refraktion sowie einer Videozentriermessung verordnet und zentriert werden.

Dabei ist von Nachteil, dass es keine Referenzierung zwischen der Refraktionsbestimmung und der Zentrierung gibt, weil beide Vorgänge mit unterschiedlichen Apparaturen durchgeführt werden. Wenn die Refraktion beispielsweise mit einem Phoropter ermittelt wird, kann es vorkommen, dass der Phoropter um einen ersten Winkel relativ zu der der Linie verkippt ist, die die beiden Pupillenmittelpunkte des Auges verbindet. Ferner kann es vorkommen, dass die gewählte Brillenfassung relativ zu dieser Linie um einen zweiten Winkel verdreht ist. Im ungünstigsten Falle können sich die beiden Winkel addieren, was zu einer Diskrepanz von mehreren Winkelgraden in den Achsen der Zylinder zwischen der Verordnung und der ausgeführten Korrektur der fertigen Brille führen kann.

Aus der WO 2006/106248 A1 sind ein Verfahren und eine Vorrichtung zum Bestimmen der Augendrehpunktlage bekannt. Bei diesem bekannten Verfahren blickt ein Proband in ein fernrohrartiges Gerät. Die Ausrichtung des Gerätes im Raum wird durch einen am Gerät befindlichen, dreidimensional wirksamen Sensor bestimmt. Der Proband trägt an seinem Kopf einen weiteren derartigen Sensor, der die Lage und Ausrichtung seines Kopfes bestimmt. In dem Gerät befindet sich auf der vom Probanden abgewandten Seite eine Lichtquelle, die einen Lichtstrahl längs einer optischen Achse ausstrahlt. Zwischen der Lichtquelle und dem Auge des Probanden befinden sich zwei Gitter mit zentralen Marken. Der Proband bewegt das Gerät nun so lange, bis sich der Lichtstrahl und die beiden Marken decken. Aus den dabei ermittelten Positionsdaten für das Gerät und den Kopf wird die Lage der Blickachse im Raum errechnet. Der Vorgang wird dann mehrfach unter unterschiedlichen Blickrichtungen wiederholt, so dass mehrere Blickachsen bestimmt werden. Deren Drehpunkt wird dann als Augendrehpunktlage berechnet.

Diese bekannte Vorgehensweise hat den Nachteil, dass ein erheblicher Aufwand an separaten Geräten betrieben werden muss. Ferner ist die Messgenauigkeit vom subjektiven Verhalten des Probanden abhängig.

Aus der EP 0 825 826 A1 sind ein Verfahren und eine Vorrichtung zum parallelen Erfassen von Sehinformationen bekannt. Zum Bestimmen der Augendrehpunktlage wird bei dieser bekannten Vorgehensweise bei fester Ausrichtung und Lage des Kopfes des Probanden mindestens für zwei bekannte, nacheinander von den Augen fixierte Fixierpunkte eine Fixierlinien-Bestimmung durchgeführt. Die Fixierpunkte sind zwei fest mit der Halterung von Kameras und Sensoren zur Bestimmung der Pupillenlage verbindbare Markierpunkte. Der Augendrehpunkt liegt dann im Schnittpunkt der durch die Fixierpunkte gelegten Fixierlinien.

Die EP 1 837 699 A1 beschreibt ein Verfahren zur Bestimmung einer progressiven ophthalmischen Linse für einen Brillenträger, der eine Fernsichtverordnung erhalten hat und für den ein Nahzusatz verschrieben worden ist. Das Verfahren umfasst unter anderem den Schritt des Bestimmens der axialen Länge des Auges des Brillenträgers. Die axiale Länge des Auges wird unter Zuhilfenahme der Fernsichtverordnung und des Radius der Hornhaut des Trägers bestimmt. Weiter wird die Augendrehpunktlage unter Zuhilfenahme der axialen Länge des Auges berechnet.

Der Erfindung liegt die Aufgabe zugrunde, andere Verfahren zur Bestimmung der Augendrehpunktlage zur Verfügung zu stellen. Ferner soll ein Verfahren bereitgestellt werden, das es möglich macht, beim Erfassen von Parametern des Systems Auge/Brille mit mehreren verschiedenen Messungen, insbesondere unter Verwendung von unterschiedlichen Geräten, eine gemeinsame räumliche Referenz für die Messwerte zu finden.

Bei dem erfindungsgemäßen Verfahren wird diese Aufgabe gelöst durch die in Anspruch 1 angegebenen Schritte.

Die mittlere Krümmung der Hornhaut bezeichnet den Mittelwert der Krümmung der Hornhaut im Bereich des Hornhautscheitels, im Allgemeinen in einem kreisförmigen Bereich mit dem Radius 4 mm um den Hornhautscheitel. Der Phasenfehler des Auges ist die Abweichung der Phase einer aus dem Auge austretenden Wellenfront von einer Referenzwelle, hier i.A. von einer ebenen Wellenfront. Der mittlere Phasenfehler bezeichnet die mittlere Krümmung der Wellenfront. Die Augenlänge ist die geometrische Länge des Auges zwischen Hornhautscheitel und der Fovea. Unter Augendrehpunktlage versteht man ganz allgemein den Ort des optischen Augendrehpunkts. Als optischer Augendrehpunkt (Kurzzeichen Z') wird z.B. nach der DIN 5340-43 der Fußpunkt des Lotes vom mechanischen Augendrehpunkt auf die ins Augeninnere verlängerte Fixierlinie beim Blick geradeaus auf einen unendlich fernen Punkt bei ungezwungener Kopf- und Körperhaltung angenommen. Der mechanische Augendrehpunkt (Kurzzeichen M) ist z.B. nach der DIN 5340 - 42 derjenige Punkt im Auge, der sich bei Blickbewegungen am wenigsten verlagert.

Die der Erfindung zugrunde liegende Aufgabe wird ferner durch eine Vorrichtung zum Durchführen des vorstehend genannten Verfahrens gelöst.

Insbesondere ist eine erste der eingangs genannten Vorrichtungen zur Bestimmung der Augendrehpunktlage in einem Auge eines Probanden gekennzeichnet durch:

a) eine Krümmungsermittlungseinrichtung zum Ermitteln der mittleren Krümmung der Hornhaut des Auges;

b) eine Phasenfehlermesseinrichtung zum Ermitteln des mittleren Phasenfehlers des Auges, wobei die mittlere Krümmung (KH) und der mittlere Phasenfehler (PF) räumlich referenziert ermittelt werden, also bezogen auf eine bestimmte Achse oder die Pupillenmitte

c) eine Augenlängenberechnungseinrichtung zum Ermitteln der Augenlänge aus der mittleren Krümmung und dem mittleren Phasenfehler; und

d) eine Augendrehpunktbestimmungseinrichtung zum Bestimmen der Augendrehpunktlage aus der Augenlänge.

Die Krümmungsermittlungseinrichtung kann z.B. ein Videokeratograph oder ein Keratometer sein. Die Phasenfehlermesseinrichtung kann z.B. als Autorefraktor oder Wellenfrontsensor ausgebildet sein. Die Augenlängenberechnungseinrichtung und die Augendrehpunktbestimmungseinrichtung können z.B. gemeinsam in Form eines handelsüblichen Personal Computers realisiert sein.

Die der Erfindung zugrunde liegende Aufgabe wird auf diese Weise vollkommen gelöst.

Das vorstehende Verfahren zeichnet sich dadurch aus, dass die Augendrehpunktlage noch exakter als herkömmlich bestimmt werden kann. Dadurch ist eine noch bessere Optimierung insbesondere von individuellen Gleitsichtgläsern möglich, wenn man die Augendrehpunktlage bei der Berechnung und Herstellung der Oberflächentopographie der individuellen Gleitsichtgläser berücksichtigt.

Die mittlere Krümmung der Hornhaut und der mittlere Phasenfehler des Auges können ohne Einschränkung an den Durchstoßpunkten einer gegebenen Achse mit den dreidimensionalen Messungen der Hornhauttopographie bzw. Wellenfrontfehler des Auges ermittelt werden.

Durch das Verfahren wird es möglich, die vorstehend genannte noch bessere Optimierung mittels einer nur geringfügigen Modifizierung bekannter Systeme zu erreichen.

Bei dem erfindungsgemäßen Verfahren kann die Augendrehpunktlage ADL aus der Augenlänge LA nach der Beziehung:

$$\mathrm{ADL} = k_3 \mathrm{LA} \qquad\qquad\qquad (1)$$

bestimmt werden. Die in Meter angegebene Größe ADL gibt dabei den Abstand zwischen dem auf der Fixierlinie liegenden Hornhautscheitel und dem Zentrum des optischen Augendrehpunkts Z' an. $k_3$ ist ein vorgebbarer, dimensionsloser Parameter.

Dieser Parameter $k_3$ kann z.B. der Theorie von Gullstrand folgend zu 13,5/23,8 gewählt werden. Abweichungen von diesem Wert von z.B. $\pm$ 10% bzw. 5% können jedoch im Allgemeinen zugelassen werden.

Bei diesem Verfahren kann die Augenlänge LA z.B. aus der mittleren Krümmung KH und dem mittleren Phasenfehler PF nach der Beziehung:

$$LA = (k_1 - PF)KH/k_2 \qquad\qquad (2)$$

bestimmt werden. Die mittlere Krümmung KH wird im Allgemeinen in Millimeter und der mittlere Phasenfehler wird im Allgemeinen in der Dimension Dioptrien angegeben. Die Parameter $k_1$ und $k_2$ sind grundsätzlich frei vorgebbar. $k_1$ hat die Dimension des Phasenfehlers, nämlich Dioptrien, $k_2$ hat ebenfalls die Dimension Dioptrien.

Folgt man einer Theorie der Erfinder so wählt man $k_1$ zu 52,634 dpt und $k_2$ zu 17,395 dpt Abweichungen von diesen Werten um $\pm$ 10% bzw. $\pm$ 5% können jedoch i. A. zugelassen werden. Diese Maßnahme hat den Vorteil, dass für den bei weitem überwiegenden Anteil der Bevölkerung eine optimale Ermittlung der Augendrehpunktlage gewährleistet ist.

In diesem Verfahren ist es im Allgemeinen ausreichend in Schritt a) die mittlere Krümmung KH der Hornhaut im Bereich der Pupillenöffnung zu ermitteln. Da die Aufweitung der Pupillenöffnung von der Umgebungshelligkeit abhängt, nimmt man üblicherweise den Bereich im Durchmesser von 8 mm um die Pupillenmitte.

Da es bei der Krümmung KH der Hornhaut und dem Phasenfehler PF des Auges besonders auf den Bereich um die optische Achse ankommt, wird im Allgemeinen in Schritt a) die mittlere Krümmung KH der Hornhaut und in Schritt b) der Phasenfehler PF jeweils im Bereich um die optische Achse und/oder im Bereich um die Fixierlinie und/oder im Bereich um die Sehachse des Auges ermittelt werden. Typische Radien um diese Achsen sind 4 mm. Es genügen im Allgemeinen auch Radien von bis zu 2 mm oder gar 1 mm. Als optische Achse des Auges kann z.B. die in DIN 5340 - vorgeschlagene Normale auf die Hornhautvorderfläche angenommen werden, deren Verlängerung in das Augeninnere von den Krümmungsmittelpunkten der übrigen brechenden Flächen des Auges den geringsten Abstand hat. Als Sehachse (Gesichtslinie) kann die nach DIN 5340 - 360 vorgeschlagene Verbindungsgerade zwischen dem zentral abgebildeten Objektpunkt und seinem Bildpunkt auf der Netzhaut verwendet werden. Als Fixierlinie kann z.B. die in DIN 5340 - 159 vorgeschlagene Verbindungsgerade zwischen dem in der Fovelamitte abgebildeten Objektpunkt und der Mitte der Eintrittspupille des Auges verwendet werden.

Diese vorstehend angegebenen Maßnahmen haben den Vorteil, dass die Ermittlung der genannten Werte verbessert wird, indem diese Werte zueinander räumlich referenziert ermittelt werden, also bezogen auf eine bestimmte Achse oder die Pupillenmitte.

Der Phasenfehler kann z.B. mittels eines Wellenfront-Autorefraktors nach dem Hartmann-Shack-Verfahren gemessen werden. Aus der mit Hilfe des Wellenfront-Autorefraktors gemessenen Phasenfehlerverteilung wird dann ein Mittelwert gebildet. Dieser Mittelwert stellt den mittleren Phasenfehler PF dar. Die Bestimmung des Phasenfehlers mit Hilfe eines Wellenfront-Autorefraktors hat den Vorteil, dass örtliche Variationen des Phasenfehlers berücksichtigt werden. Anstelle eines Wellenfront-Autorefraktors kann auch ein Autorefraktometer verwendet werden.

Ein Computerprogramm mit Programmcode kann zur Durchführung des vorstehend beschriebenen Verfahrens eingerichtet sein, wenn das Programm in einem Computer einer Vorrichtung ausgeführt wird, welche eine Krümmungsermittlungseinrichtung, eine Phasenfehlermesseinrichtung, eine Augenlängenberechnungseinrichtung und eine Augendrehpunktbestimmungseinrichtung umfasst. Das Computerprogramm kann z.B. auf einem maschinenlesbaren Datenträger gespeichert sein.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:

Figur 1    eine erste schematische Seitenansicht eines nach oben verdrehten Auges zur Erläuterung von verschiedenen Blickrichtungen und Drehpunkten und der Lage des Augendrehpunktes;

Figur 2    eine zweite schematische Seitenansicht eines geradeaus gerichteten Auges mit vorgesetztem Brillenglas zur Erläuterung weiterer Parameter des Auges;

Figur 3    eine Frontansicht eines Auges zur Erläuterung bestimmter Augenbereiche;

Figur 4    eine dritte schematische Seitenansicht eines geradeaus gerichteten Auges zur Erläuterung der Messung des Phasenfehlers des Auges mit Hilfe eines Wellenfront-Autorefraktors;

Figur 5    ein Diagramm, wie es im Rahmen des ersten Verfahrens der vorliegenden Erfindung zur Ermittlung der Augendrehpunktlage verwendet werden kann;

Figur 6    eine Vorrichtung zur Bestimmung der Augendrehpunktlage in einem Auge eines Probanden nach der Erfindung;

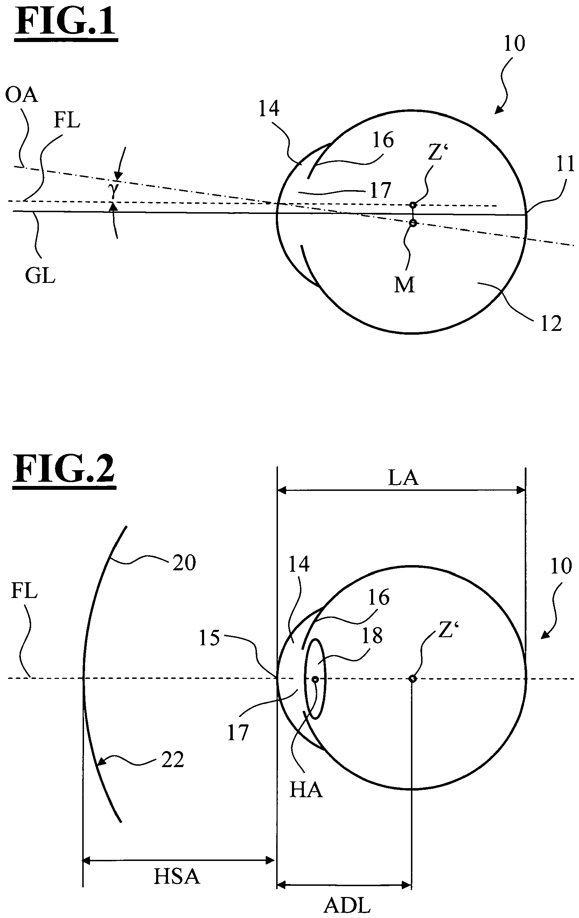

In den Figuren 1 und 2 bezeichnet 10 ein Auge. Das Auge 10 hat einen Glaskörper 12, eine Hornhaut 14, eine Iris 16, eine Pupille 17 sowie eine Linse 18.

Wenn das Auge 10 eine Drehbewegung ausführt, dann geschieht dies nicht exakt um einen Drehpunkt im Raum. Vielmehr gibt es nur einen näherungsweise kugelförmigen Bereich, in dem sich die momentanen Drehpunkte befinden. Derjenige Punkt, der bei Augenbewegungen die geringste Lageveränderung erfährt, wird als mechanischer Augendrehpunkt M bezeichnet (vgl. DIN 5340 - 42).

Mit GL ist die Sehachse (Gesichtslinie) bezeichnet. Sie ist nach DIN 5340 - 360 die Verbindungsgerade zwischen einem fixierten Objektpunkt und dem dazu konjugierten Bildpunkt in der Mitte der Netzhautgrube 11.

Mit FL ist die Fixierlinie (Visierlinie) bezeichnet. Sie ist nach DIN 5340 - 159 die Verbindungsgerade zwischen dem zentral abgebildeten Objektpunkt und der Mitte der Eintrittspupille 17.

OA bezeichnet die optische Achse.

Mit Z' ist der optische Augendrehpunkt bezeichnet. Er ist nach DIN 5340 - 43 der Fußpunkt des Lotes vom mechanischen Augendrehpunkt M auf die Fixierlinie FL.

Der Winkel zwischen der optischen Achse OA und der Fixierlinie FL ist in Figur 1 mit $\gamma$ bezeichnet. Der Winkel $\gamma$ ist hier nur in einer Ebene eingezeichnet, symbolisiert aber einen Raumwinkel oben/unten und /rechts/links.

In Figur 2 ist ein Brillenglas 20 vor dem Auge 10 angeordnet. Das Brillenglas hat auf der dem Auge 10 zugewandten Seite eine Rückfläche 22. Der Abstand der Rückfläche 22 von dem Hornhaut-Apex 15 gemessen in Blickrichtung senkrecht zur Fassungsebene wird als Hornhaut-Scheitelabstand HSA bezeichnet (vgl. DIN EN ISO 13666 - 5.27). Der Abstand des Hornhautscheitels 15 vom optischen Augendrehpunkt Z' gibt die Augendrehpunktlage ADL in Bezug auf den Hornhaut-Apex 15 an.

Die Augendrehpunktlage ADL ist ein wichtiger Parameter in der Berechnung des Brillenglases 20. Das Brillenglas 20 wird immer so optimiert, dass es für jede Blickrichtung des Auges 10 die optimalen Abbildungseigenschaften aufweist.

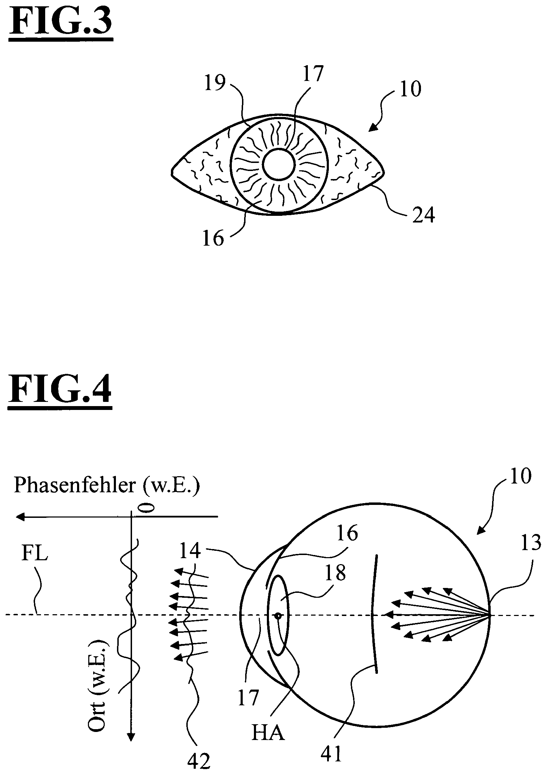

Figur 3 zeigt eine Frontansicht des Auges 10. Man erkennt in der Iris 17 eine charakteristische Struktur sowie neben der Iris 17 in der Lederhaut 24 eine Struktur von kleinen Blutgefäßen.

Ausführungsbeispiel:

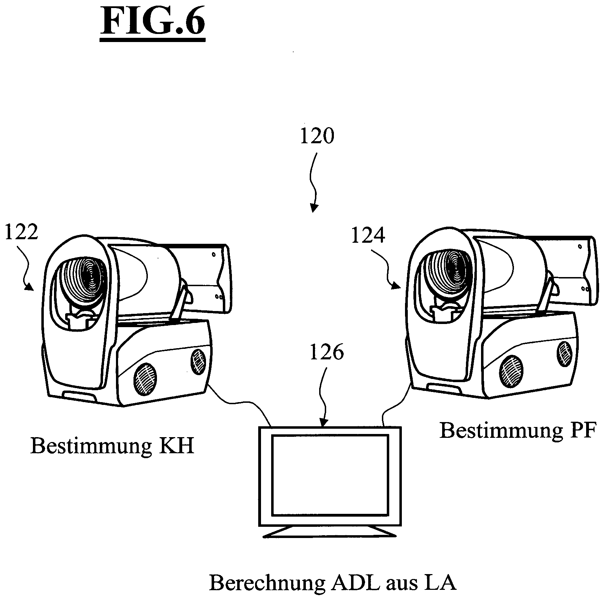

Bei einem Ausführungsbeispiel eines erfindungsgemäßen Verfahrens wird die Augendrehpunktlage ADL auf der Grundlage eines Augenmodells und einer Abschätzung der Augenlänge LA ermittelt. Eine erfindungsgemäße Vorrichtung 120 zur Durchführung des Verfahrens zeigt die Figur 6. Das Verfahren umfasst folgende Schritte:

In einem ersten Schritt 1a) wird mit einem Gerät 122, nämlich mittels eines geeigneten Scanners die Topographie der Hornhaut 14 und daraus die mittlere Krümmung KH der Hornhaut 14 ermittelt. Bei dieser Messung kann zugleich die Lage der Mitte der Pupille 17 sowie zusätzlich die Lage des Scheitels der Hornhaut 14 ermittelt werden.

In einem zweiten Schritt 1b) werden die Phasenfehler und der Mittelwert des Phasenfehlers PF des Auges 10 ermittelt. Hierzu bedient man sich beispielsweise eines Wellenfront-Autorefraktors 124 bekannter Bauart, der die Verteilung der Phasenfehler über die gesamte Öffnung der Pupille 17 des Auges 10 ermittelt, wie dies in der Figur 4 dargestellt ist. Die Phasenfehlerverteilung kann Beispielsweise mit dem sogenannten Standard Hartmann-Shack-Verfahren bestimmt werden. Dieses Verfahren basiert auf dem Vergleich einer an der Netzhaut 13 gestreuten und durch das Auge hindurch getretenen Wellenfront 42 mit der Wellenfront 41 vor der Streuung und dem Augendurchtritt. Dabei kann auch die Lage der Mitte der Pupille 17 ermittelt werden. Die beiden Messungen können ggf. auch mit zwei unterschiedlichen Messanordnungen in einem Gerät durchgeführt werden (aus diesem Grund zeigt die Figur 6 zwei identische Geräte 122, 124). Ein Computer 126 errechnet dann aus der Verteilung der Phasenfehler über dem Ort den arithmetischen Mittelwert des Phasenfehlers PF des Auges 10 des Probanden.

Um in den vorgenannten beiden Schritten ein gemeinsames Bezugssystem für die gemessenen Lagen herzustellen, kann erfindungsgemäß zusätzlich in jedem der beiden Schritte 1a) und 1b) eine photographische Aufnahme hergestellt werden, die beispielsweise die in Figur 3 dargestellten Strukturen der Iris 17 oder der Blutgefäße in der Lederhaut 24 erfasst. Diese Strukturen können dann als Referenzsystem für die Lagen der Pupillenmitte und des Hornhautscheitels sowie weiterer Parameter verwendet werden.

Aus den so ermittelten Werten KH und PF wird nun in einem dritten Schritt 1c) die Augenlänge LA bestimmt. Hierzu kann man sich der Beziehung

$$LA = (k_1 - KR)KH/k_2$$

bedienen, deren Konstanten $k_1$ und $k_2$ aus einem Modell gewonnen werden, das durch Messungen an einer Vielzahl von Augen erstellt wird/worden ist.

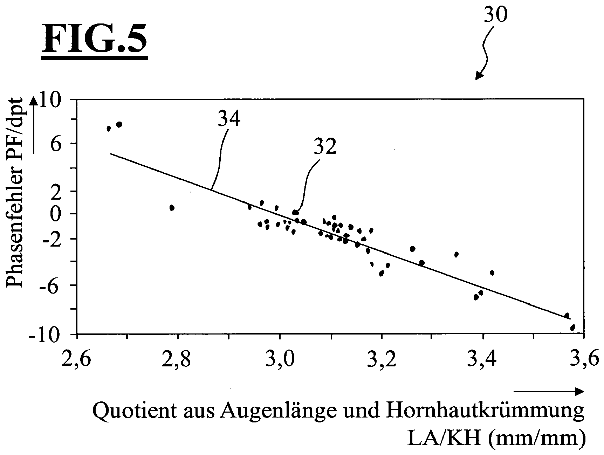

Figur 5 zeigt hierzu ein Diagramm 30 mit dem Zusammenhang zwischen dem Quotienten aus Augenlänge und Hornhautkrümmung (Abszisse LA/KH) und des Phasenfehlers des Auges (Ordinate PF). Die Punkte bezeichnen die Ergebnisse realer Messungen an Testpersonen. Die Ausgleichsgerade 34 gibt den linearen Zusammenhang zwischen den Messwerten wieder.

Aus dem Verlauf der Geraden 34 kann man die Konstanten $k_1$ und $k_2$ wie folgt entnehmen:

$$k_1 = 52,634 \text{ dpt}$$

$$k_2 = 17,395 \text{ dpt}$$

Mit der so bestimmten Augenlänge LA kann man in einem vierten Schritt 1d) die Augendrehpunktlage ADL nach der Beziehung

$$ADL = k_3 LA$$

ermitteln, wobei z.B. nach Gullstrand als Erfahrungswert gilt:

$$k_3 = 13,5/23,8$$

Die derart bestimmte Augendrehpunktlage ADL kann als Eingangsparameter in der Berechnung eines individuell optimierten Brillenglases verwendet werden.

Bevorzugt wird in Schritt a) die mittlere Krümmung KH der Hornhaut 14 im Bereich der Pupillenöffnung ermittelt. Alternativ oder zusätzlich kann in Schritt a) die mittlere Krümmung KH der Hornhaut 14 und in Schritt b) der Phasenfehler PF jeweils im Bereich um eine Achse OA, GL des Auges 10 ermittelt werden. Die mittlere Krümmung KH der Hornhaut 14 wird dabei insbesondere in einem Bereich mit dem Durchmesser 12 mm um den Hornhautscheitel ermittelt, der mittlere Phasenfehler PF in einem entsprechenden Bereich um die Pupillenmitte. Die beiden Werte werden auf diese Weise räumlich referenziert, also z.B. beide auf eine bestimmte charakteristische Achse OA oder GL oder die Pupillenmitte bezogen.

Bezugszeichenliste

| 10 | Auge |
| 11 | Netzhautgrube |
| 12 | Glaskörper |
| 13 | Netzhautebene |
| 14 | Hornhaut |
| 15 | Hornhautscheitel |
| 16 | Iris |
| 17 | Pupille |
| 18 | Linse |
| 19 | Limbus |

| 20 | Brillenglas |
|----|----|
| 22 | Rückfläche |
| 24 | Lederhaut |
| 30 | Diagramm |
| 32 | Messpunkt |
| 34 | Gerade |
| 41 | Wellenfront |
| 42 | Wellenfront |
| 120 | Vorrichtung zur Bestimmung der Augendrehpunklage |
| 122 | iProfiler |
| 124 | iProfiler |
| 126 | Computer |
| ADL | Augendrehpunktlage |
| HA | objektseitiger Hauptpunkt des Auges |
| HSA | Hornhaut-Scheitelabstand |
| FL | Fixierlinie |
| GL | Sehachse |
| PF | mittlerer Phasenfehler |
| KH | Krümmung der Hornhaut |
| LA | Augenlänge |
| M | mechanischer Augendrehpunkt |
| OA | optische Achse |
| Z' | optischer Augendrehpunkt |
| x, y, z | Raumkoordinaten |
| $\gamma$ | Winkel,Blickrichtung |

## Patentansprüche

1. Verfahren zur Bestimmung der Augendrehpunktlage (ADL) in einem eine Pupille (17) und eine Pupillenmitte aufweisenden Auge (10) eines Probanden mit den Schritten:

    a) Ermitteln der mittleren Krümmung (KH) der Hornhaut (16) des Auges (10), mittels einer Krümmungsermittlungseinrichtung (122);
    b) Ermitteln des mittleren Phasenfehlers (PF) des Auges (10), mittels einer Phasenfehlermesseinrichtung (126); wobei die mittlere Krümmung (KH) und der mittlere Phasenfehler (PF) räumlich referenziert ermittelt werden, also bezogen auf eine bestimmte Achse oder die Pupillenmitte
    c) Ermitteln der Augenlänge (LA) aus der mittleren Krümmung (KH) und dem mittleren Phasenfehler (PF) mittels einer Augenlängenberechnungseinrichtung (126); und
    d) Bestimmen der Augendrehpunktlage (ADL) aus der Augenlänge (LA) mittels einer Augendrehpunktbestimmungseinrichtung (126).

2. Verfahren nach Anspruch 1, **dadurch gekennzeichnet, dass** die Augendrehpunktlage (ADL) aus der Augenlänge (LA) nach der Beziehung:

$$\text{ADL} = k_3 \text{LA}$$

bestimmt wird.

3. Verfahren nach Anspruch 2, **dadurch gekennzeichnet, dass**

$$k_3 = 13\ 5/23\ 8 \pm 10\%$$

ist.

4. Verfahren nach einem der vorangegangenen Ansprüche, **dadurch gekennzeichnet, dass** die Augenlänge (LA) aus der mittleren Krümmung (KH) und dem mittleren Phasenfehler (PF) nach der Beziehung:

$$LA = (k_1 - PF)KH/k_2$$

bestimmt wird.

5. Verfahren nach Anspruch 4, **dadurch gekennzeichnet, dass**

$$k_1 = 52{,}634 \text{ dpt} \pm 10\%$$

$$k_2 = 17{,}395 \text{ dpt} \pm 10\%$$

ist.

6. Verfahren nach einem der vorangegangenen Ansprüche, **dadurch gekennzeichnet, dass** in Schritt a) die mittlere Krümmung (KH) der Hornhaut (16) im Bereich der Pupillenöffnung ermittelt wird.

7. Verfahren nach einem der vorangegangenen Ansprüche, **dadurch gekennzeichnet, dass** in Schritt a) die mittlere Krümmung (KH) der Hornhaut (16) und in Schritt b) der Phasenfehler (PF) jeweils im Bereich um die optische Achse (OA) und/oder die Fixierlinie (FL) und/oder die Sehachse (GL) des Auges (10) ermittelt werden.

8. Verfahren nach einem der vorangegangenen Ansprüche, **dadurch gekennzeichnet, dass** der Phasenfehler (PF) mittels eines Wellenfront-Autorefraktors gemessen wird.

9. Verfahren zum Optimieren eines für ein Auge (10) eines Probanden individuellen Brillenglases (20), bei dem die Augendrehpunktlage (ADL) ermittelt und als Eingangsparameter verwendet wird, **dadurch gekennzeichnet, dass** zur Bestimmung der Augendrehpunktlage (ADL) in dem Auge (10) des Probanden ein Verfahren nach einem der vorangegangenen Ansprüche verwendet wird.

10. Computerprogramm mit Programmcode eingerichtet zur Durchführung des Verfahrens nach einem der vorangegangenen Ansprüche, wenn das Programm in einem Computer einer Vorrichtung ausgeführt wird, welche eine Krümmungsermittlungseinrichtung (122), eine Phasenfehlermesseinrichtung (126), eine Augenlängenberechnungseinrichtung (126) und eine Augendrehpunktbestimmungseinrichtung (126) umfasst.

11. Computerprogramm nach Anspruch 10, gespeichert auf einem maschinenlesbaren Datenträger.

12. Vorrichtung (120) zur Bestimmung der Augendrehpunktlage (ADL) in einem eine Pupille (17) und eine Pupillenmitte aufweisenden Auge (10) eines Probanden mit:

a) einer Krümmungsermittlungseinrichtung (122) zum Ermitteln der mittleren Krümmung (KH) der Hornhaut (16) des Auges (10),
b) einer Phasenfehlermesseinrichtung (126) zum Ermitteln des mittleren Phasenfehlers (PF) des Auges (10); wobei die mittlere Krümmung (KH) und der mittlere Phasenfehler (PF) räumlich referenziert ermittelt werden, also bezogen auf eine bestimmte Achse oder die Pupillenmitte
c) einer Augenlängenberechnungseinrichtung (126) zum Ermitteln der Augenlänge (LA) aus der mittleren Krümmung (KH) und dem mittleren Phasenfehler (PF); und
d) einer Augendrehpunktbestimmungseinrichtung (126) zum Bestimmen der Augendrehpunktlage (ADL) aus der Augenlänge (LA).

**Claims**

1. Method for determining the location of the eye fulcrum (ADL) in an eye (10), having a pupil (17) and a pupil centre, of a test subject, having the steps of:

a) determining the mean curvature (KH) of the cornea (16) of the eye (10), by means of a curvature determination device (122);
b) determining the mean phase error (PF) of the eye (10), by means of a phase error measurement device (126); wherein the mean curvature (KH) and the mean phase error (PF) are determined in a spatially referenced manner, i.e. in relation to a particular axis or the pupil centre;
c) determining the eye length (LA) from the mean curvature (KH) and the mean phase error (PF) by means of an eye length calculation device (126); and
d) determining the location of the eye fulcrum (ADL) from the eye length (LA) by means of an eye fulcrum determination device (126).

2. Method according to Claim 1, **characterized in that** the location of the eye fulcrum (ADL) is determined from the eye length (LA) by using the relationship:

$$\mathrm{ADL} = \mathrm{k}_3\mathrm{LA}.$$

3. Method according to Claim 2, **characterized in that**

$$\mathrm{k}_3 = 13\ 5/23\ 8 \pm 10\%.$$

4. Method according to one of the preceding claims, **characterized in that** the eye length (LA) is determined from the mean curvature (KH) and the mean phase error (PF) by using the relationship:

$$\mathrm{LA} = (\mathrm{k}_1-\mathrm{PF})\mathrm{KH}/\mathrm{k}_2.$$

5. Method according to Claim 4, **characterized in that**

$$\mathrm{k}_1 = 52.634\ \mathrm{dpt} \pm 10\%$$

$$\mathrm{k}_2 = 17.395\ \mathrm{dpt} \pm 10\%.$$

6. Method according to one of the preceding claims, **characterized in that** in step a) the mean curvature (KH) of the cornea (16) in the region of the pupil opening is determined.

7. Method according to one of the preceding claims, **characterized in that** in step a) the mean curvature (KH) of the cornea (16) is determined, and in step b) the phase error (PF) is respectively determined in the region around the optical axis (OA), the fixation line (FL) and/or the visual axis (GL) of the eye (10) are determined.

8. Method according to one of the preceding claims, **characterized in that** the phase error (PF) is measured by means of a wavefront autorefractor.

9. Method for optimizing a spectacle lens (20) customized for an eye (10) of a test subject, in which the location of the eye fulcrum (ADL) is determined and used as input parameter, **characterized in that** a method according to one of the preceding claims is used in determining the location of the eye fulcrum (ADL) in the eye (10) of the test subject.

10. Computer program with program code set up to carry out the method according to one of the preceding claims when the program is executed in a computer of an apparatus which comprises a curvature determination device (122), a phase error measurement device (126), an eye length calculation device (126) and an eye fulcrum determination device (126).

**11.** Computer program according to Claim 10, stored on a machine-readable data medium.

**12.** Apparatus (120) for determining the location of the eye fulcrum (ADL) in an eye (10), having a pupil (17) and a pupil centre, of a test subject, having:

> a) a curvature determination device (122) for determining the mean curvature (KH) of the cornea (16) of the eye (10);
> b) a phase error measurement device (126) for determining the mean phase error (PF) of the eye (10); wherein the mean curvature (KH) and the mean phase error (PF) are determined in a spatially referenced manner, i.e. in relation to a particular axis or the pupil centre;
> c) an eye length calculation device (126) for determining the eye length (LA) from the mean curvature (KH) and the mean phase error (PF); and
> d) an eye fulcrum determination device (126) for determining the location of the eye fulcrum (ADL) from the eye length (LA).

**Revendications**

**1.** Procédé de détermination de la position du foyer oculaire (ADL) dans un oeil (10) d'un sujet clinique qui possède une pupille (17) et un centre de pupille, comprenant les étapes suivantes :

> a) détermination de la courbure moyenne (KH) de la cornée (16) de l'oeil (10) au moyen d'un dispositif de détermination de la courbure (122) ;
> b) détermination de l'erreur de phase moyenne (PF) de l'oeil (10) au moyen d'un dispositif de mesure de l'erreur de phase (126) ;
> la courbure moyenne (KH) et l'erreur de phase moyenne (PF) étant déterminées avec référence dans l'espace, c'est-à-dire rapportées à un axe donné ou au centre de la pupille
> c) détermination de la longueur de l'oeil (LA) à partir de la courbure moyenne (KH) et de l'erreur de phase moyenne (PF) au moyen d'un dispositif de calcul de la longueur de l'oeil (126) ; et
> d) détermination de la position du foyer oculaire (ADL) à partir de la longueur de l'oeil (LA) au moyen d'un dispositif de détermination de la position du foyer oculaire (126).

**2.** Procédé selon la revendication 1, **caractérisé en ce que** la position du foyer oculaire (ADL) est déterminée à partir de la longueur de l'oeil (LA) d'après la relation :

$$ADL = k_3 LA.$$

**3.** Procédé selon la revendication 2, **caractérisé en ce que**

$$k_3 = 13\ 5/23\ 8 \pm 10\ \%.$$

**4.** Procédé selon l'une des revendications précédentes, **caractérisé en ce que** la longueur de l'oeil (LA) est déterminée à partir de la courbure moyenne (KH) et de l'erreur de phase moyenne (PF) d'après la relation :

$$LA = (k_1 - PF)KH/k_2.$$

**5.** Procédé selon la revendication 4, **caractérisé en ce que**

$$k_1 = 52{,}634\ dtp \pm 10\ \%$$

$$k_2 = 17,395 \text{ dtp} \pm 10 \%.$$

6. Procédé selon l'une des revendications précédentes, **caractérisé en ce qu'**à l'étape a), la courbure moyenne (KH) de la cornée (16) est déterminée dans la zone de l'ouverture de la pupille.

7. Procédé selon l'une des revendications précédentes, **caractérisé en ce que** la courbure moyenne (KH) de la cornée (16) à l'étape a) et l'erreur de phase (PF) à l'étape b) sont respectivement déterminées dans la zone autour de l'axe optique (OA) et/ou de la ligne visuelle (FL) et/ou de l'axe visuel (GL) de l'oeil (10).

8. Procédé selon l'une des revendications précédentes, **caractérisé en ce que** l'erreur de phase (PF) est mesurée au moyen d'un autoréfracteur à front d'onde.

9. Procédé d'optimisation d'un verre de lunette (20) individuel pour un oeil (10) d'un sujet clinique, avec lequel la position du foyer oculaire (ADL) est déterminée et utilisée comme paramètre d'entrée, **caractérisé en ce qu'**un procédé selon l'une des revendications précédentes est utilisé pour déterminer la position du foyer oculaire (ADL) dans l'oeil (10) du sujet clinique.

10. Programme informatique comprenant un code de programme conçu pour mettre en oeuvre le procédé selon l'une des revendications précédentes lorsque le programme est exécuté dans un ordinateur d'un dispositif qui comprend un dispositif de détermination de la courbure (122), un dispositif de mesure de l'erreur de phase (126), un dispositif de calcul de la longueur de l'oeil (126) et un dispositif de détermination de la position du foyer oculaire (126).

11. Programme informatique selon la revendication 10, enregistré sur un support de données lisibles par machine.

12. Dispositif (120) de détermination de la position du foyer oculaire (ADL) dans un oeil (10) d'un sujet clinique qui possède une pupille (17) et un centre de pupille, comprenant :

a) un dispositif de détermination de la courbure (122) destiné à déterminer la courbure moyenne (KH) de la cornée (16) de l'oeil (10) ;
b) un dispositif de mesure de l'erreur de phase (126) destiné à déterminer l'erreur de phase moyenne (PF) de l'oeil (10) ; la courbure moyenne (KH) et l'erreur de phase moyenne (PF) étant déterminées avec référence dans l'espace, c'est-à-dire rapportées à un axe donné ou au centre de la pupille ;
c) un dispositif de calcul de la longueur de l'oeil (126) destiné à déterminer la longueur de l'oeil (LA) à partir de la courbure moyenne (KH) et de l'erreur de phase moyenne (PF) ; et
d) un dispositif de détermination de la position du foyer oculaire (126) destiné à déterminer la position du foyer oculaire (ADL) à partir de la longueur de l'oeil (LA).

## FIG.1

## FIG.2

# FIG.3

# FIG.4

# FIG.5

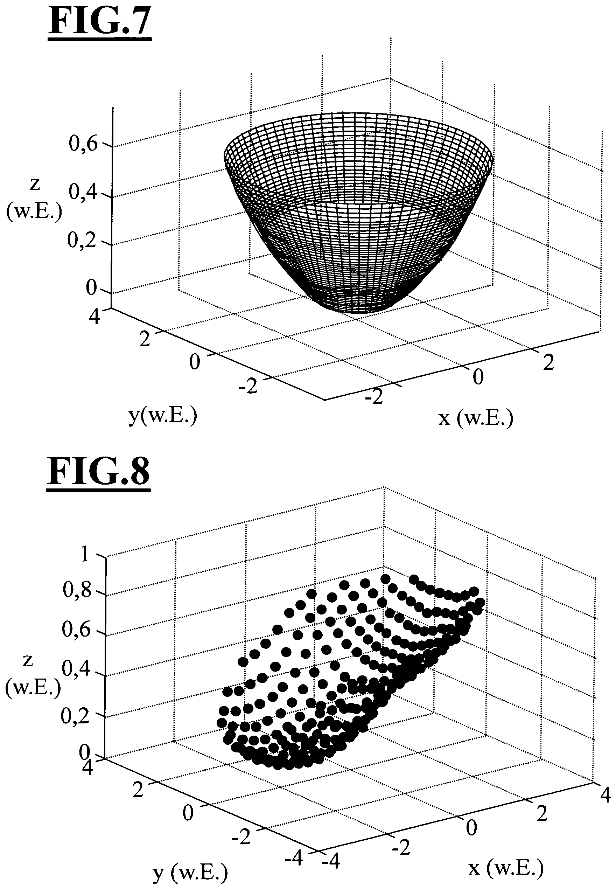

## FIG.7

## FIG.8

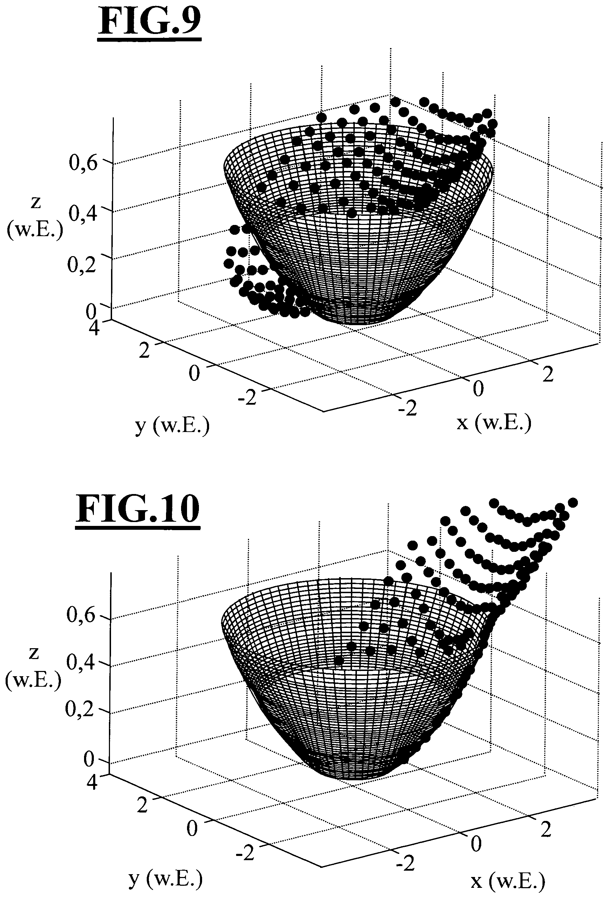

## FIG.9

## FIG.10

# FIG.6

Bestimmung KH

Berechnung ADL aus LA

Bestimmung PF

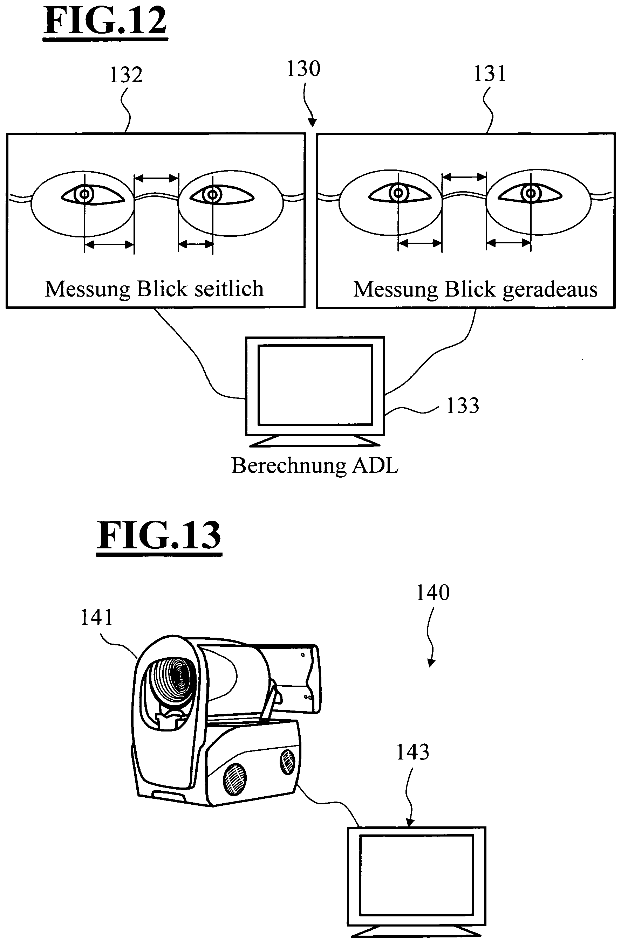

# FIG.12

132  130   131

Messung Blick seitlich  Messung Blick geradeaus

133

Berechnung ADL

# FIG.13

140

141

143

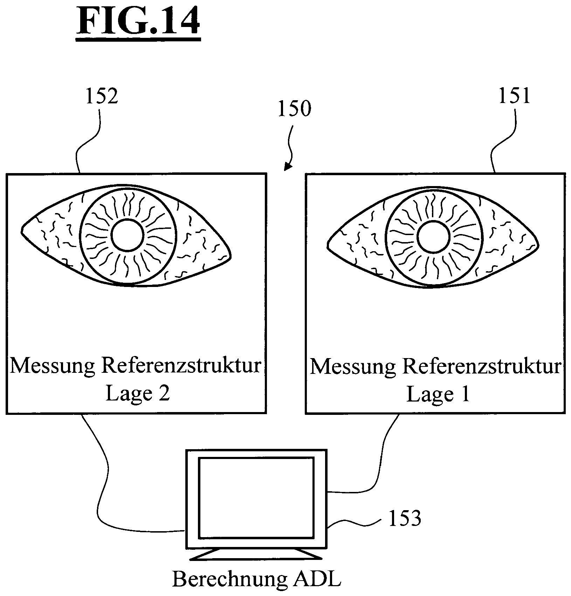

# FIG.14

152    150      151

Messung Referenzstruktur
Lage 2

Messung Referenzstruktur
Lage 1

153

Berechnung ADL

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 857993 B1
- EP 562336 B1
- WO 2006106248 A1
- EP 0825826 A1
- EP 1837699 A1